(19) Europäisches Patentamt
European Patent Office
Office européen des brevets

(11) Publication number: **0 315 981 B1**

(12) **EUROPEAN PATENT SPECIFICATION**

(45) Date of publication of patent specification: **14.07.93**

(51) Int. Cl.[5]: **B01D 71/64**, B01D 53/22, C08G 73/10

(21) Application number: **88118686.0**

(22) Date of filing: **09.11.88**

The file contains technical information submitted after the application was filed and not included in this specification

# (54) Process for separating methane using permeable membrane.

(30) Priority: **12.11.87 JP 286789/87**
**23.12.87 JP 327843/87**
**23.12.87 JP 327844/87**
**23.02.88 JP 41657/88**
**20.04.88 JP 97739/88**
**30.09.88 JP 248534/88**

(43) Date of publication of application:
**17.05.89 Bulletin 89/20**

(45) Publication of the grant of the patent:
**14.07.93 Bulletin 93/28**

(84) Designated Contracting States:
**DE FR GB IT NL**

(56) References cited:
**EP-A- 0 143 552**
**DE-B- 2 849 978**
**GB-A- 2 051 664**
**GB-A- 2 073 654**

**PATENT ABSTRACTS OF JAPAN vol. 8, no.212 (C-244)(1648), 27th September 1984**

(73) Proprietor: **NITTO DENKO CORPORATION**
**1-2, Shimohozumi 1-chome Ibaraki-shi Osaka(JP)**

(72) Inventor: **Shimatani, Shunichi NITTO DENKO CORPORATION**
**1-2, Shimohozumi 1-chome**
**Ibaraki-shi Osaka(JP)**
Inventor: **Yamamoto, Michiharu NITTO DENKO CORPORATION**
**1-2, Shimohozumi 1-chome**
**Ibaraki-shi Osaka(JP)**
Inventor: **Shimazu, Akira NITTO DENKO CORPORATION**
**1-2, Shimohozumi 1-chome**
**Ibaraki-shi Osaka(JP)**
Inventor: **Iwama, Akio NITTO DENKO CORPORATION**
**1-2, Shimohozumi 1-chome**
**Ibaraki-shi Osaka(JP)**

(74) Representative: **Patentanwälte Grünecker, Kinkeldey, Stockmair & Partner**
**Maximilianstrasse 58**
**W-8000 München 22 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**EP 0 315 981 B1**

Rank Xerox (UK) Business Services
(3.10/3.6/3.3.1)

## Description

This invention relates to a process for separating methane by concentration using a selective permeable membrane. More particularly, it relates to a process for effectively separating methane from a gaseous mixture containing methane and carbon dioxide by the use of a membrane having selective permeability to carbon dioxide.

Separation of methane by concentration of a gaseous mixture containing methane and carbon dioxide, such as natural gas, off-gas of oil field gas, blast furnace gas, burning furnace gas, etc., by means of a permeable membrane has been generally conducted at high tempratures above 50 ° C. These gaseous mixtures mostly contain acidic gases, such as, e.g., sulfur dioxide or hydrogen sulfide, in addition to carbon dioxide. Thefefore, the membrane to be used for separation of methane from such a gaseous mixture is required not only to have selective permeability to carbon dioxide but also to have heat resistance, acid resistance, hydrocarbon resistance as well as mechanical strength.

Conventional membranes for separation of methane include composite membranes mainly comprising cellulose resins, e.g., cellulose acetate and ethyl cellulose. However, these membranes comprising cellulose resins are inferior in heat resistance and hydrocarbon resistance and it has been difficult, therefore, to stably separate methane by the use of these conventional membranes.

EP-A-0 143 552 discloses a composite membrane suitable for use in membrane separation of an organic solution containing a relatively small solute, liquid/liquid separation be the pervaporation method, and concentration and separation of a gas mixture such as the separation of oxygen from nitrogen in air. More specifically, this document discloses a polyamide ultrafiltration membrane used in the preparation of a composite membrane used for the separation of various gases.

A process for preparing a selective permeable membrane made from polyimides which are suitable for use in the membraneseparation treatment of organic liquid mixtures as well as for aqueous liquid mixtures is known from GB-A-2 051 664. This document, however, does not disclose membranes suitable for separating gaseous mixtures.

JP-A-5998704 wants to provide a heat resistant separation membrane comprising polimide for gas separation, particularly for oxygen enrichment of air. Therein the separation coefficient of $CO_2$ to $N_2$ is disclosed only.

It is the object of this invention to provide a process for separating methane by concentration by using a membrane excellent in permeability to carbon dioxide, heat resistance, acid resistance, hydrocarbon resistance, and mechanical strength.

According to the present invention this object is attained with process for separating methane from a gaseous mixture containing methane and carbon dioxide by concentration, which comprises contacting said gaseous mixture with a membrane comprising film of a polyimide resin having a repeating unit represented by formula (I):

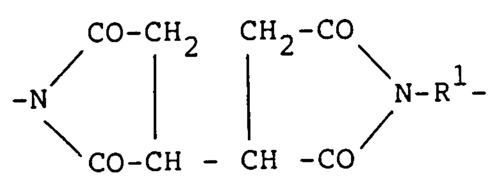

(I)

wherein $R^1$ represents a divalent aromatic, alicyclic or aliphatic hydrocarbon group, or a divalent organic group composed of these hydrocarbon groups linked via a divalent organic linking group, to selectively pass carbon dioxide through said membrane.

According to a preferred embodiment the present invention relates to process for separating methane from a gaseous mixture containing methane and carbon dioxide by concentration, which comprises contacting said gaseous mixture with a composite membrane composed of the above-described polyimide resin membrane as a supporting film and a film comprising an elastomeric polymer to selectively pass carbon dioxide through said composite membrane. Further preferred embodiments of the present invention are set out in the dependent claims attached hereto.

The membrane comprising the polyimide resin serves as a selective carbon dioxide-permeable membrane by itself and also as a supporting film of a composite membrane.

The membrane comprising the polyimide resin is known per se as described, e.g., in U.S. Patents 4,240,914 and 4,358,378.

In formula (I), $R^1$ represents a divalent aromatic, alicyclic or aliphatic hydrocarbon group, or a divalent organic group composed of these hydrocarbon groups linked via a divalent organic linking group.

The divalent aromatic hydrocarbon group preferably includes phenylene groups having from 6 to 12 carbon atoms. The organic linking group which connects such divalent aromatic hydrocarbon groups to form a divalent organic group includes, e.g., an alkylene group (e.g., methylene, isopropylidene), an ether group, a sulfido group, a sulfo group, an amido group, an ester group, a urethane group and a urea group.

The divalent aliphatic hydrocarbon group preferably includes straight chain or branched alkylene groups having from 1 to 10 carbon atoms. The organic linking group which connects such divalent aliphatic hydrocarbon groups to form a divalent organic group includes, e.g., an ether group, a sulfido group, an amido group, an ester group, a urethane group, a urea group and a polyoxyalkylene group.

The divalent alicyclic hydrocarbon group preferably includes cyclohexylene groups having from 6 to 12 carbon atoms and alkyl-substituted cyclohexylene groups. The organic linking group which connects such divalent alicyclic hydrocarbon groups to form a divalent organic group includes, e.g., an alkylene group (e.g., methylene, isopropylidene), an ether group, a sulfido group, a sulfo group, an amido group, an ester group, a urethane group and a urea group.

From the standpoint of heat resistance and hydrocarbon resistance, the polyimide resin preferably includes those wherein $R^1$ represents an aromatic hydrocarbon group or an aromatic hydrocarbon group composed of two aromatic hydrocarbon groups connected by an organic linking group, such as, e.g., an alkylene group (e.g., methylene, isopropylidene), an ether group, a sulfide group or a sulfo group, more preferably those wherein $R^1$ represents an aromatic hydrocarbon group composed of two aromatic hydrocarbon groups connected via an alkylene group (e.g., methylene, isopropylidene) or an ether group, such

as 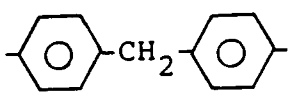 and 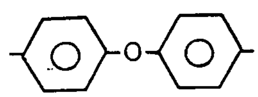.

Also preferred is the polyimide resin of formula (I) wherein $R^1$ represents a divalent organic group containing a sulfo group. Examples of such a divalent organic group are groups represented by formula (II):

$-R^2-SO_2-R^3-$ (II)

wherein $R^2$ and $R^3$ each represents a divalent aromatic, alicyclic or aliphatic hydrocarbon group, or a divalent organic group composed of these hydrocarbon groups via a divalent organic linking group.

In formula (II), the divalent aromatic hydrocarbon group as represented by $R^2$ or $R^3$ preferably includes phenylene groups having from 6 to 12 carbon atoms, and the organic linking group which connects such divalent aromatic hydrocarbon groups to form a divalent organic group includes an alkylene group (e.g., methylene, isopropylidene), an ether group, and a sulfido group.

The divalent aliphatic hydrocarbon group as represented by $R^2$ or $R^3$ preferably includes straight chain or branched alkylene groups having from 1 to 10 carbon atoms, and the organic linking group which connects such divalent aliphatic hydrocarbon groups to form a divalent organic group includes an ether group, a sulfido group, and a polyoxyalkylene group.

The divalent alicyclic hydrocarbon group as represented by $R^2$ or $R^3$ preferably includes cyclohexylene groups having from 6 to 12 carbon atoms and alkyl-substituted cyclohexylene groups, and the organic linking group which connects such divalent alicyclic hydrocarbon groups to form a divalent organic group includes a methylene group, an isopropylidene group, an ether group, and a sulfido group.

From the standpoint of heat resistance and hydrocarbon resistance, more preferred of the polyimide resins containing a divalent organic group of formula (II) are those wherein both $R^2$ and $R^3$ represent an aromatic hydrocarbon group or an aromatic hydrocarbon group composed of two aromatic hydrocarbon groups linked via an organic linking group, such as an alkylene group (e.g., methylene, isopropylidene), an ether group, and a sulfido group.

Specific examples of the most preferred groups of formula (II) are

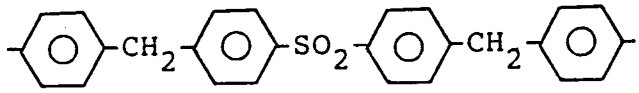,

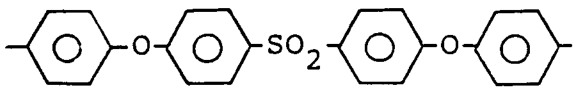, and 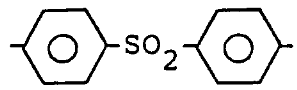.

In order that these polyimide resins may exhibit excellent film-forming properties in the wet film formation process hereinafter described and also that the resulting film may have sufficient mechanical strength, it is desirable that the resins have an intrinsic viscosity [$\eta$] of from 0.4 to 2 d$\ell$/g, more preferably from 0.4 to 1 d$\ell$/g, as measured in N-methyl-2-pyrrolidone at 30 °C (hereinafter the same).

When a gaseous mixture containing methane and carbon dioxide is brought into contact with the above-stated polyimide resin having a sulfo group in the molecule thereof as a separating membrane, a very high rate of permeation to carbon dioxide is achieved. It is assumed to be because the solubility of carbon dioxide in the membrane is enhanced by the sulfo group of the resin.

The polyimide resin membrane to be used as a selective permeable membrane by itself or as a supporting membrane of a composite membrane can preferably be prepared in accordance with the following wet process.

A film forming solution comprising the polyimide resin having the repeating unit of formula (I) and a water-miscible organic solvent (hereinafter referred to as a first organic solvent) is prepared. The solution is coated on an appropriate base and then dipped in an organic solvent incapable of dissolving the polyimide resin but miscible with either of the first organic solvent and water (hereinafter referred to as a second organic solvent) for a short time. Thereafter, the film on the base is dipped in water to obtain a water-containing polyimide membrane having an anisotropic structure, which is then dried by an appropriate means to thereby obtain a membrane having an anisotropic structure having a so-called skin layer.

Specific examples of the first organic solvent to be used in the film-forming solution include an N-alkyl-2-pyrrolidone (e.g., N-methyl-2-pyrrolidone), an N-alkyl-2-piperidone (e.g., N-methyl-2-piperidone), a dialkylacetamide (e.g., dimethylacetamide), and a dialkylformamide (e.g., dimethylformamide), with N-methyl-2-pyrrolidone being preferred.

The concentration of the polyimide resin in the film-forming solution usually ranges from 10 to 40% by weight, preferably from 15 to 30% by weight. If it is too high, the excessive viscosity of the solution makes it difficult to uniformly apply the solution on a base and, in addition, the resulting membrane has poor permeability to carbon dioxide for practical use. In connection with the polyimide resin concentration, the viscosity of the film-forming solution is usually adjusted to a range of from 5 x $10^{-2}$ to 500 x $10^{-2}$ Pa•s (50 to 5,000 poise), preferably from 1 x $10^{-2}$ to 200 x $10^{-2}$ Pa•s (100 to 2,000 poise).

The film-forming solution is coated on the base to a thickness usually of from 50 to 400 $\mu$m, preferably from 100 to 300 $\mu$m. Further, a thin film only produces a membrane failing to have a practical strength. If the film thickness is too large, the resulting membrane is of no utility due to a low rate of gas permeation, though high in separation selectivity.

The base to be used for film formation includes sheets having a smooth surface, such as, e.g., a glass plate, a stainless steel sheet or a resin sheet, and a woven or non-woven cloth. Membranes obtained by using woven or non-woven cloth as a base are advantageous because of enhanced strength.

Specific examples of the second solvent incapable of dissolving the polyimide resin but miscible with either of the first organic solvent and water include, e.g., lower aliphatic alcohols (e.g., methanol, ethanol, propanol, butanol), alcohols having a cyclic structure (e.g., furfuryl alcohol, tetrahydrofurfuryl alcohol), ketones (e.g., acetone, methyl ethyl ketone, diacetone alcohol), esters of formic acid, acetic acid, lactic acid and phosphoric acid; polyhydric alcohols (e.g., ethylene glycol, propylene glycol, dipropylene glycol, trimethylene glycol, butanediol, glycerin, pentanediol, hexylene glycol, diethylene glycol, triethylene glycol, tetraethylene glycol) and derivatives thereof such as ethers and esters (e.g., methyl cellosolve, cellosolve acetate, butyl cellosolve, methylcarbitol, butylcarbitol, carbitol acetate, dipropylene glycol monomethyl ether, butoxyethoxypropanol, propylene glycol monomethyl ether, triethylene glycol monomethyl ether);

ethers (e.g., dioxane, tetrahydrofuran); and nitriles (e.g., acetonitrile). These solvents may be used either individually or in combinations of two or more thereof.

The dipping in the second organic solvent is usually effected at a temperature of from 0° to 100°C for 1 h or less, preferably 5 min or less.

After the film is dipped in the second organic solvent, it is dipped in water usually at a temperature of from 0 to 50°C, preferably from 0° to 30°C, more preferably from 0° to 20°C, to obtain a water-containing membrane having anisotropy.

Thereafter, the resulting water-containing membrane is dipped in an organic solvent having miscibility with either water and an organic solvent, e.g., alcohols, and then in an organic solvent immiscible with water, e.g., hexane, followed by drying at room temperature or, if necessary, under heating to obtain a dried membrane possessing selective gas permeability. Other methods for drying the water-containing membrane are also applicable.

The thus obtained membrane can be used as it is for separation of methane by concentration. The membrane can also be used as a supporting membrane on which a thin film of an elastomeric polymer is formed.

In a preferred embodiment of the above-described wet film formation process, the film-forming solution further contains a liquid swelling agent. A membrane prepared therefrom has further increased permeability to carbon dioxide.

The liquid swelling agent which can be used in this preferred embodiment is at least one liquid organic compound having a flocculation value for the polyimide resin according to the present invention ranging from 50 to 200 and having a boiling point of from 50 to 120°C at atmospheric pressure which is selected from the group consisting of cyclic ethers, aliphatic ketones, alicyclic ketones, lower aliphatic carboxylic acids, and lower aliphatic carboxylic acid lower alkyl esters.

The teminology "flocculation value of a swelling agent for the polyimide resin" as used herein means a minimum number of milliliters of the swelling agent necessary to make 50 mℓ of a 2 wt% solution of the polyimide resin in N-methylpyrrolidone become white turbid at 25°C due to flocculation of the resin.

The swelling agent should be soluble in both of the first organic solvent and water and have a flocculation value for the polyimide resin of from 50 to 200 and a boiling point of from 50 to 120°C at atmospheric pressure.

Specific and preferred examples of such a swelling agent are tetrahydrofuran, dioxane, acetone, methyl ethyl ketone, diethyl ketone, cyclohexanone, acetic acid, formic acid, methyl formate, ethyl formate, and methyl acetate. The swelling agent is added to the film-forming solution in an amount of from 30 to 300 parts by weight, preferably from 50 to 150 parts by weight, per 100 parts by weight of the polyimide resin. A large amount of the swelling agent sometimes hinders uniformity of the film-forming solution too. If the amount of the swelling agent is too small, effects as expected cannot be produced.

The swelling agent can be incorporated into the film-forming solution, for example, by previously adding it to a solution of the polyimide resin and stirring the mixture at room temperature or, if necessary, under heating.

The membrane prepared from the film-forming solution containing the above-described swelling agent is the most preferred for use as a selective permeable membrane either by itself or in combination with an elastomeric polymer film for separation of methane by concentration.

In the former case, when a gaseous mixture containing methane and carbon dioxide is brought into contact with the selective permeable membrane, the carbon dioxide selectively passes through the membrane to thereby obtain a gaseous mixture comprising concentrated methane in the side to which the gaseous mixture to be treated is fed and a gaseous mixture comprising concentrated carbon dioxide in the side into which carbon dioxide passes. If desired, the gaseous mixture containing methane in high concentration can be subjected to further processing, such as compression, adsorption, condensation and the like, to separate methane therefrom.

The polyimide resin membrane according to the present invention essentially has a high permeability to carbon dioxide and a coefficient of $CO_2/CH_4$ separation as high as 100 or even more. However, when it is formed into a thin film suited for separation of methane by concentration, the membrane sometimes unavoidably suffers from defects or pinholes. Therefore, it is not always easy to stably obtain a thin membrane having such a high coefficient of separation on an industrial scale.

In this connection, it is preferable in the present invention that the membrane comprising the polyimide resin is used as a supporting membrane on which a thin film of an elastomeric polymer is provided to obtain a composite membrane for separation of methane. That is, the composite film can achieve separation of methane from a gaseous mixture containing carbon dioxide with great advantages at an extremely high coefficient of $CO_2/CH_4$ separation while stably retaining a high rate of permeation to carbon dioxide.

The polyimide resin membrane serving as a support for the composite membrane preferably has a coefficient of $CO_2/CH_4$ separation of at least 0.7, and particularly at least 0.9.

The composite membrane according to the present invention can be produced by forming a thin film of an elastomeric polymer on the dense skin layer of the polyimide resin supporting membrane.

The elastomeric polymer which can be used is a polymer capable of forming a soft film. Typical examples of such an elastomeric polymer include homo-or copolymers of ethylenically unsaturated monomers or conjugated diene monomers, such as polypropylene, polyvinyl chloride, an ethylene-propylene copolymer, an ethylene-propylene-diene copolymer, polybutadiene, polyisoprene, chloroprene rubber, poly(4-methyl-penten-1), a butadiene-styrene copolymer, an isoprene-isobutylene copolymer, and polyisobutylene. Copolymers comprising the above-enumerated monomers and compolymerizable monomers having a functional group, such as acrylonitrile, (meth)acrylic esters, and (meth)acrylic acid, can also be used preferably. Preferred among these polymers are homo- or copolymers of ethylenically unsaturated monomers or conjugated diene monomers.

In addition, silicone resins are also preferred as an elastomeric polymer. In particular, a film obtained by crosslinking a crosslinkable silicone resin is one preferred elastomeric polymer film.

Further implicit in the elastomeric polymer to be used in the composite membrane are copolymers having both soft segments and hard segments, such as polyester polyol, polyurethane polyether, polyurethane polyester, and polyamide polyether. Specific examples of such copolymers are shown below:

1) 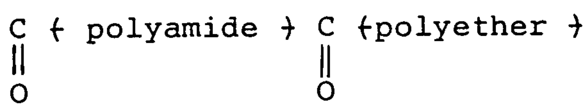

(hard segment) (soft segment)

2) 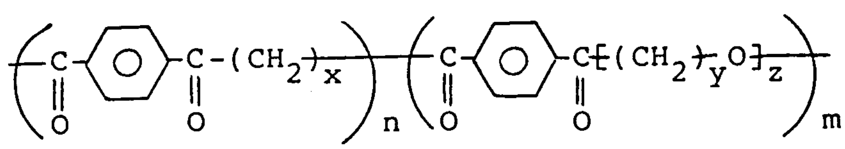

(hard segment) (soft segment)

[x: 1 to 4; y: 1 to 10; z: 1 to 100; n, m: degree of polymerization of from 10 to 10,000]

3)

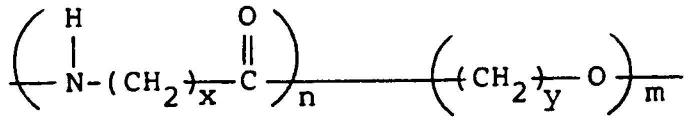

(hard segment) (soft segment)

[x: 1 to 5; n, m: degree of polymerization of from 10 to 10,000]

4)

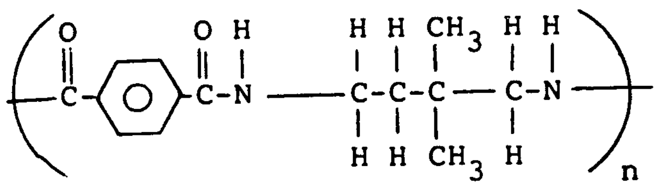

(hard segment) (soft segment)

[n: degree of polymerization of from 10 to 10,000]

Furthermore, epoxy resins curable by a straight long chain curing agent, ethyl cellulose, and butoxy resins are also employable as elastomeric polymer.

The above-described elastomeric polymers having introduced into the molecular chain or terminals thereof a functional group, e.g., a carboxyl group, a hydroxyl group, a hydroxyalkyl group, an amido group, an amino group, an alkoxy group, etc. are also preferred.

These elastomeric polymers preferably have a coefficient of $CO_2/CH_4$ separation of at least 3 and a softening point of at least 50 °C, particularly at least 80 °C.

The composite membrane according to the present invention can be obtained by dissolving the elastomeric polymer in an appropriate organic solvent and coating the solution on the dense skin layer of the supporting membrane comprising the polyimide resin, if necessary curing the coating film, and drying.

The elastomeric polymer solution usually has a concentration of from 0.05 to 10% by weight, preferably from 0.1 to 5% by weight. The elastomeric polymer solution is coated on the supporting membrane to a thickness of from 0.01 to 5 $\mu$m, preferably from 0.05 to 1 $\mu$m. The coating means includes, for example, an applicator and a microgravure coater. The coating may be carried out by dipping the supporting membrane in the elastomeric polymer solution.

In the present invention, it is preferable that the elastomeric polymer on the polyimide supporting membrane is crosslinked by an appropriate means. Namely, the composite membrane according to the invention preferably comprises the polyimide resin supporting membrane having formed thereon a crosslinked elastomeric polymer film.

The gaseous mixture containing methane generally contains petroleum hydrocarbons, and in most cases, aliphatic hydrocarbons having 3 or more carbon atoms that are approximately the same with crude oil. By the use of the composite membrane having the crosslinked elastomeric polymer film, methane can efficiently be separated from a gaseous mixture without involving swelling of the membrane or a reduction in separation performance due to the petroleum hydrocarbons even in long-term treatment.

Crosslinking of the elastomeric polymer film on the supporting membrane can be carried out typically by using a crosslinking agent or irradiating an electron beam on the elastomeric polymer film.

The process using a crosslinking agent is effected by coating a solution containing the elastomeric polymer and a crosslinking agent on the surface of the supporting membrane and then heating the elastomeric polymer film to induce crosslinking. It is essential that the crosslinking agent should not cause a crosslinking reaction of the elastomeric polymer during the coating until heated. If a crosslinking agent working at room temperature is used, the elastomeric polymer precipitates in the coating solution or undergoes gelation due to crosslinking during coating, thus failing to form a thin and uniform membrane excellent in gas permeability.

Included in the crosslinking agent satisfying the above-stated requirement are, for example, radical generators, e.g., benzoyl peroxide, methyl ethyl ketone peroxide, cumene hydroperoxide, azobisisobutyronitrile, etc.

When the elastomeric polymer contains a functional group, e.g., a carboxyl group, a hydroxyl group, a hydroxyalkyl group, an amido group, an amino group or an alkoxy group, in the molecule thereof or at the terminals thereof as mentioned above, a compound having a polyfunctional group capable of reacting with such a functional group, e.g., polyisocyanate, is preferably used as a crosslinking agent.

In addition, when the elastomeric polymer contains a carboxyl group, it may be ion-crosslinked in the presence of a polyvalent metallic ion, e.g., aluminum, calcium, magnesium, copper, barium or zinc ion. In this case, too, the polyvalent metallic ion to be used should be selected from those which do not cause crosslinking at room temperature but are reacted with the functional group of the elastomeric polymer upon heating.

Radiation to be used for crosslinking the elastomeric polymer film may be any ionizing radiation and includes, for example, electron rays, neutron rays, $\alpha$-rays, $\beta$-rays, $\gamma$-rays, and ultraviolet rays. The irradiation dose usually ranges from $10^4$ to 50 x $10^4$ J/kg (1 to 50 Mrad) preferably from 3 x $10^4$ to 20 x $10^4$ J/kg (3 to 20 Mrad) though varying depending on the temperature, pressure, etc. of the irradiation atmosphere.

The irradiation induces radical generation in the side chain of the polymer, and the radical molecules are mutually crosslinked to form a crosslinked film on the supporting membrane.

Whichever crosslinking process may be selected, the degree of crosslinking should be properly selected so as not to cause swelling or dissolution of the crosslinked film with petroleum hydrocarbons present in the gaseous mixture to be treated and is suitably determined in accordance with the kind of the elastomeric polymer or the crosslinking process adopted. Excessive crosslinking would rather impair properties of the elastomeric polymer, causing cracks of the resulting composite membrane.

The elastomeric polymer solution has a polymer concentration usually of from 0.05 to 10% by weight, preferably from 0.1 to 5% by weight. The solution is usually coated on the supporting membrane to a film thickness of from 0.01 to 5 $\mu$m, preferably from 0.05 to 1 $\mu$m. The coating means includes an applicator, a microgravure coater, etc. The coating may be effected by dipping the supporting membrane in the elastomeric polymer solution.

The composite membrane having a crosslinked silicone resin as a crosslinked elastomeric polymer can be obtained by crosslinking a crosslinkable silicone resin film formed on the supporting membrane. The crosslinkable silicone resin is a silicone resin which is soluble in organic solvents before crosslinking but provides an organic solvent-insoluble resin on crosslinking. Such a crosslinkable silicone resin carries various reactive groups at each of the molecular chain terminals, by which the molecules are mutually crosslinked in the presence or absence of a crosslinking agent or a curing agent.

More specifically, the crosslinkable silicone resin which can be used in the present invention is generally represented by formula:

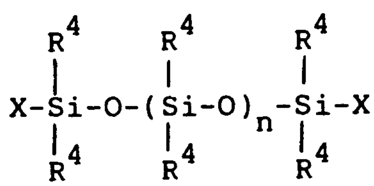

or formula:

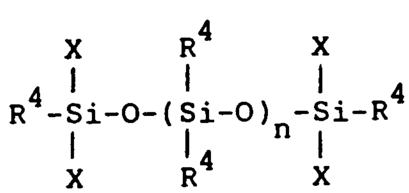

wherein X represents a reactive group; and $R^4$ represents an alkyl or aryl group; and a part of the repeating unit may be replaced by a group represented by formula:

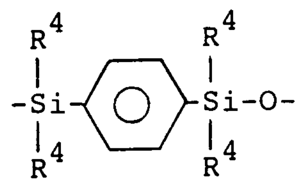

wherein $R^4$ is as defined above.

In the above formulae, the reactive group as represented by X includes a vinyl group, an acryloxyalkyl group, a methacryloxyalkyl group, a hydroxyl group, a hydroxyalkyl group, an acyloxy group, an alkoxy group, an aminoalkyl group, a carboxylalkyl group, a ketoxime group, an alkylamino group, an amido group, etc. A part of $R^4$ may be replaced with such a reactive group. $R^4$ typically includes a methyl group and a phenyl group.

Various kinds of the crosslinkable silicone resin having the above-enumerated reactive groups are already known and are available as commercial products.

If desired, crosslinking of the crosslinkable silicone resin having reactive groups at the molecular terminals thereof may be carried out in the presence of a crosslinking agent, a curing agent, or a polymerization initiator. For example, when a vinyl-terminated organopolysiloxane or (meth)acryloxyalkyl-terminated organopolysiloxane is used, a radical generator is employed as a crosslinking agent. Examples of such a radical generator are organic peroxides, e.g., benzoyl peroxide, methyl ethyl ketone peroxide, and cumene hydroperoxide, and azo compounds, e.g., azobisisobutyronitrile. When crosslinkable silicone resins having, e.g., a hydroxyl group, a hydroxyalkyl group, an acyloxy group, an alkoxy group, an alkylamino group, an amido group or a ketoxime group as reactive group are used, crosslinking can be induced by the water content in the atmosphere. When crosslinkable silicone resins having, e.g., an aminoalkyl group, a carboxyalkyl group, a hydroxyl group or a hydroxyalkyl group as reactive group are used, a bi- or polyfunctional polyisocyanate or an epoxy resin is preferably used as a curing agent. The polyisocyanate includes, e.g., tolylene diisocyanate, m-xylylene diisocyanate, 4,4'-diphenylmethane diisocyanate, polytetramethylene glycol diisocyanate, polypropylene glycidyl diisocyanate, etc. When crosslinkable silicone resins having an acyloxy group, an alkoxy group, a ketoxime group, an alkylamino group and an amido group as a reactive group are used, crosslinking can easily be induced by polyhydric alcohols. The polyhydric alcohols include, e.g., ethylene glycol, propylene glycol, butylene glycol, pentanediol, hexanediol, diethylene glycol, dipropylene glycol, triethylene glycol and glycerin. Of these polyhydric alcohols, glycerin or triethylene glycol are particularly useful since they can induce crosslinking of the crosslinkable silicone resin at ambient temperature without requiring heating. The polyhydric alcohol may be incorporated into the solution of the crosslinkable silicone resin as in the case of one-can curable resins.

If an organic solution of the crosslinkable silicone resin applied onto the skin layer of the supporting membrane penetrates into the inside of the supporting membrane, a dense crosslinked silicone resin is formed also on the inside of the polyimide membrane, thereby providing a dense layer having an increased thickness. To this effect, it is desirable that the crosslinkable silicone resin to be used be chosen so as to have an average molecular weight equal to or larger than the fractional molecular weight of the polyimide supporting membrane. Usually, a crosslinkable silicone resin having an average molecular weight of from 10,000 to 300,000 as determined by gel-permeation chromatography is preferred. The crosslinkable silicone resin having such a molecular weight range is implicit in the silicone resins having the above-described formula wherein n is between 100 and 4,000.

The organic solvent to be used for dissolving the crosslinkable silicone resin can be selected, appropriately, depending on the kind of the resin, from, for example, aliphatic hydrocarbons, aromatic hydrocarbons, alcohols, carboxylic acids, esters, ketones, ethers and halogenated hydrocarbons.

The solution of the crosslinkable silicone resin usually has a concentration of from 0.05 to 10% by weight, preferably from 0.1 to 5% by weight and is coated on the supporting memberane to a film thickness of from 0.01 to 5 µm, preferably from 0.05 to 1 µm. The coating can be carried out by means of, e.g. an applicator or a microgravure coater or by dipping the supporting membrane in the crosslinkable silicone resin solution.

If necessary or depending on the kind of the organic solvent used, the solvent is removed at an appropriate temperature, for example, at room temperature up to about 80 °C. The crosslinkable silicone resin is then crosslinked in the presence or absence of a crosslinking agent, a curing agent or a radical initiator to form a three-dimensional structure. If necessary, the crosslinking temperature may be higher than

the above-recited temperature.

The thus formed crosslinked silicone resin film preferably has a coefficient of $CO_2/CH_4$ separation of at least 3.

For the purpose of increasing the crosslinking density, the crosslinked silicone resin film may be irradiated with ionizing radiation, such as electron rays, neutron rays, $\alpha$-rays, $\beta$-rays, $\gamma$-rays, ultraviolet rays, etc. The irradiation dose usually ranges from $10^4$ - 50 x $10^4$ J/kg (1 to 50 Mrad), preferably from 3 x $10^4$ - 20 x $10^4$ J/kg (3 to 20 Mrad), though varying depending on the temperature or pressure of the atmosphere. By this irradiation, radicals are produced in the side chains of the crosslinked silicone resin, and these radical molecules are mutually crosslinked to thereby increase the crosslinking density, which leads to further improvements on mechanical strength, resistance to organic solvents, and selective permeability.

The thus obtained composite membrane comprising the polyimide membrane and an elastomeric polymer film can be used as a selective permeable membrane for separation of methane in the same manner as described for the polyimide membrane. If desired, the resulting gaseous mixture containing concentrated methane may be subjected to further processing, such as compression, adsorption, condensation and the like, to separate methane therefrom.

The selective permeable membrane according to the present invention is usually and preferably used in the form of a so-called spiral membrane module in which the web of the membrane is spirally wound. The membrane is also used as a module having a hollow yarn structure or other modular structure.

Since the polyimide resin membrane or a composite membrane using the polyimide resin membrane as a support is excellent not only in selective permeability to carbon dioxide but also in resistance to heat and hydrocarbons, the present invention makes it poossible to stably separate methane from a gaseous mixture containing carbon dioxide together with methane.

The present invention is now illustrated in greater detail by way of the following Examples and Comparative Examples. In these examples, all the percents and parts are by weight unless otherwise indicated.

EXAMPLE 1

A 16% N-methyl-2-pyrrolidone solution of polyimide having a repeating unit of formula:

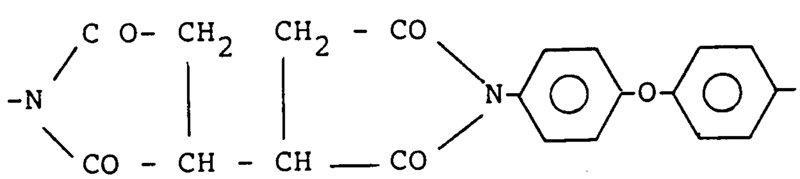

(intrinsic viscosity [$\eta$]: 0.79 dℓ/g) was coated on a polyester non-woven cloth to a thickness of 200 $\mu$m. The coated cloth was dipped in an organic solvent shown in Table 1 under indicated conditions and then in water at 3 °C to obtain a water-containing membrane having an anisotropic structure (Sample Nos. 101 to 120)

Each of the resulting water-containing membranes was soaked first in ethanol and then in hexane for 3 hours, respectively, followed by air-drying at 25 °C to obtain a dried membrane.

A rate of permeation to carbon dioxide and a coefficient of $CO_2/CH_4$ separation of the membrane were determined, and the results obtained are shown in Table 1.

TABLE 1

| Sample No. | Solvent Dipping Conditions: Organic Solvent | Temp. (°C) | Time (sec) | Rate of Permeation to Carbon Dioxide (Nm3/m2·hr·atm) | Nm³/m²·s·MPa | Coefficient of CO2/CH4 Separation |
|---|---|---|---|---|---|---|
| 101 | isopropyl alcohol | 3 | 10 | $1.9 \times 10^{-1}$ | 67.1 | 40 |
| 102 | " | " | 60 | $0.8 \times 10^{-1}$ | 28.2 | 70 |
| 103 | " | 20 | 10 | $1.4 \times 10^{-1}$ | 49.4 | 42 |
| 104 | " | " | 60 | $0.5 \times 10^{-1}$ | 17.7 | 82 |
| 105 | ethylene glycol | 3 | 10 | $4.1 \times 10^{-1}$ | 144.7 | 15 |
| 106 | " | " | 60 | $4.6 \times 10^{-1}$ | 162.4 | 14 |
| 107 | " | 20 | 10 | $6.3 \times 10^{-1}$ | 222.4 | 3 |
| 108 | " | " | 60 | $5.7 \times 10^{-1}$ | 201.2 | 15 |
| 109 | tetrahydrofuran | 3 | 10 | $11 \times 10^{-1}$ | 388.3 | 12 |
| 110 | " | " | 60 | $11 \times 10^{-1}$ | 388.3 | 11 |
| 111 | " | 20 | 10 | $11 \times 10^{-1}$ | 388.3 | 15 |
| 112 | " | " | 60 | $12 \times 10^{-1}$ | 423.6 | 14 |
| 113 | acetone | 3 | 10 | $8.8 \times 10^{-1}$ | 310.6 | 12 |
| 114 | " | " | 60 | $4.9 \times 10^{-1}$ | 173.0 | 23 |
| 115 | " | 20 | 10 | $6.8 \times 10^{-1}$ | 240.0 | 17 |
| 116 | " | " | 60 | $5.2 \times 10^{-1}$ | 183.6 | 30 |
| 117 | t-butanol | 25 | 10 | $2.7 \times 10^{-1}$ | 95.3 | 42 |
| 118 | " | " | 60 | $1.9 \times 10^{-1}$ | 67.1 | 53 |
| 119 | " | 45 | 3 | $2.2 \times 10^{-1}$ | 77.7 | 51 |
| 120 | " | " | 10 | $1.4 \times 10^{-1}$ | 49.4 | 41 |

EXAMPLE 2

A dried polyimide resin membrane was prepared in the same manner as in Example 1, except that the polyimide solution further contained a prescribed amount of a swelling agent as shown in Table 2 below.

The rate of permeation to carbon dioxide and a coefficient of $CO_2/CH_4$ separation of each of the resulting membranes (Sample Nos. 201 to 212) were determined, and the results obtained are shown in Table 2.

TABLE 2

| Sample No. | Swelling Agent: Kind | Swelling Agent: Flocculation Value | Amount Added (part*) | Rate of Permeation to Carbon Dioxide (Nm3/m2.hr.atom) | Nm³/m²·s·MPa | Coefficient of CO2/CH4 Separation |
|---|---|---|---|---|---|---|
| 201 | acetone | 63 | 25 | 1.9 x $10^{-1}$ | 67.1 | 41 |
| 202 | " | " | 50 | 2.2 x $10^{-1}$ | 77.7 | 43 |
| 203 | " | " | 100 | 1.1 x $10^{-1}$ | 38.8 | 21 |
| 204 | " | " | 200 | 1.4 x $10^{-1}$ | 49.4 | 33 |
| 205 | dioxane | 170 | 25 | 2.5 x $10^{-1}$ | 88.3 | 55 |
| 206 | " | " | 50 | 2.7 x $10^{-1}$ | 95.3 | 52 |
| 207 | " | " | 100 | 3.3 x $10^{-1}$ | 116.5 | 66 |
| 208 | " | " | 200 | 4.4 x $10^{-1}$ | 155.3 | 50 |
| 209 | tetrahydrofuran | 88 | 25 | 4.9 x $10^{-1}$ | 173.0 | 21 |
| 210 | " | " | 50 | 6.3 x $10^{-1}$ | 222.4 | 44 |
| 211 | " | " | 100 | 4.7 x $10^{-1}$ | 166.0 | 25 |
| 212 | " | " | 200 | 6.8 x $10^{-1}$ | 240.0 | 37 |

Note: * per 100 parts by weight of the polyimide resin

EXAMPLE 3

To the same polyimide resin solution as used in Example 1 were added 50 parts of dioxane (flocculation value: 170) as a swelling agent per 100 parts of the polyimide resin to form a film-forming solution.

The solution was coated to a polyester non-woven cloth to a thickness of 200 µm. The coated cloth was dipped in isopropanol at 3 °C for 10 s and then in water at 3 °C to obtain a water-containing membrane having an anisotropic structure.

The resulting water-containing membrane was soaked first in ethanol and then in hexane for 3 h respectively, followed by air-drying at 25°C to obtain a dried membrane.

On the dried membrane was coated a solution of an elastomeric polymer shown in Table 3 below to a thickness of 30 μm, followed by dring at 80°C for 30 min to prepare a composite membrane (Sample Nos. 301 to 304).

The rate of permeation to carbon dioxide and a coefficient of $CO_2/CH_4$ separation of the resulting composite membrane are shown in Table 3.

TABLE 3

| Sample No. | Elastomeric Polymer Solution: Elastomeric Polymer | Elastomeric Polymer Solution: Solvent | Elastomeric Polymer Solution: Concn. (wt%) | Coefficient of $CO_2/CH_4$ Separation of Elastomeric Polymer | Composite Membrane: Rate of Permeation to Carbon Dioxide ($Nm^3/m^2 \cdot hr \cdot atm$) $Nm^3/m^2 \cdot s \cdot MPa$ | Composite Membrane: Coefficient of $CO_2/CH_4$ Separation |
|---|---|---|---|---|---|---|
| 301 | poly(4-methyl-pentene-1) | cyclo-hexane | 1.0 | 5.4 | $2.2 \times 10^{-1}$ 77.7 | 141 |
| 302 | butadiene-styrene copolymer | toluene | 1.0 | 18 | $2.0 \times 10^{-1}$ 70.6 | 113 |
| 303 | polyisobutylene | isooctane | 0.5 | 11 | $1.0 \times 10^{-1}$ 35.3 | 73 |
| 304 | isoprene-isobutylene copolymer | toluene | 1.0 | 12 | $2.5 \times 10^{-1}$ 88.3 | 44 |

EXAMPLE 4

A composite membrane (Sample Nos. 401 to 406) was obtained in the same manner as in Example 3, except that the elastomeric polymer solution shown in Table 4 was used. In the preparation of Sample Nos.

405 and 406, the conditions for film formation on the supporting membrane were altered according to the kind of the elastomeric polymer as described in the footnote of Table 4.

The rate of permeation to carbon dioxide and the coefficient of $CO_2/CH_4$ separation of each of the resulting composite membranes are shown in Table 4.

TABLE 4

| Sample No. | Elastomeric Polymer Solution | | | Coefficient of $CO_2/CH_4$ Separation of Elastomeric Polymer | Composite Membrane | |
|---|---|---|---|---|---|---|
| | Elastomeric Polymer | Solvent | Concn. (wt%) | | Rate of Permeation to Carbon Dioxide ($Nm^3/m^2 \cdot hr \cdot atom$) [handwritten: $Nm^3/m^2 \cdot s \cdot MPa$] | Coefficient of $CO_2/CH_4$ Separation |
| 401 | polyester polyol | chloroform | 1.0 | 10 | $0.5 \times 10^{-1}$ [handwritten: 17.7] | 74 |
| 402 | polyurethane polyol | tetra-hydrofuran | 1.0 | 11 | $2.1 \times 10^{-1}$ [handwritten: 74.1] | 61 |
| 403 | polyurethane polyester | " | 1.0 | 19 | $0.8 \times 10^{-1}$ [handwritten: 28.2] | 80 |
| 404 | polyamide polyether | chloroform | 0.5 | 9 | $1.7 \times 10^{-1}$ [handwritten: 60.0] | 105 |
| 405 | epoxy resin* | toluene | 5.0 | 15 | $0.2 \times 10^{-1}$ [handwritten: 7.1] | 114 |
| 406 | ethyl cellulose** | chloroform | 2.0 | 18 | $1.0 \times 10^{-1}$ [handwritten: 35.3] | 99 |

Note: * : Comprised 100 parts of Epon 828® (made by Shell Chemical), 10 parts of a curing agent CIBN (carboxyl-terminated butadiene-acrylonitrile copolymer), and 5 parts of tetramethylguanidine; Curing Conditions: heating at 180°C for 1 hour.

**: Heated at 60°C for 30 minutes.

EXAMPLE 5

The same polyimide resin solution as used in Example 1 was prepared, and 50 parts of dioxane (flocculation value: 170) were dissolved therein as a swelling agent per 100 parts of the polyimide resin to prepare a film-forming solution.

The solution was coated on a polyester non-woven cloth to a thickness of 200 µm. The coated cloth was dipped first in isopropanol at 3°C for 10 s and then in water at 3°C to obtain a water-containing membrane having an anisotropic structure.

The water-containing membrane was soaked successively in ethanol and then in hexane for 3 h, respectively, followed by air-drying at 25°C to prepare a supporting membrane.

An elastomeric polymer solution containing a radical generator or polyisocyanate as a crosslinking agent as shown in Table 5 was coated on the supporting membrane to a thickness of 30 µm and dreid at 120°C for 10 min to obtain a composite membrane having a crosslinked elastomeric polymer film (Sample Nos. 501 to 503).

Each of the resulting composite membranes was determined for rate of permeation to carbon dioxide [P($CO_2$)] and coefficient of $CO_2/CH_4$ separation ($\alpha$), and the results obtained are shown in Table 6.

The composite membrane was immersed in petroleum benzine at room temperature for about 30 h and then in gasoline for about 30 h. After washing with hexane, the membrane was dried. P($CO_2$) and $\alpha$ of the thus treated composite membrane were determined, and the results are shown in Table 6.

For comparison, composite membranes (Samples A to C) were prepared in the same manner as Samples 501 to 503, except that the elastomeric polymer solution contained no crosslinking agent. P($CO_2$) and $\alpha$ of these comparative composite membranes were determined before and after immersion in solvents in the same manner as described above, and the results obtained are also shown in Table 6.

TABLE 5

Elastomeric Polymer Solution

| Sample No. | Elastomeric Polymer | Solvent | Concn. (wt%) | Crosslinking Agent (Amount: part*1) | |
|---|---|---|---|---|---|
| 501 | polyisobutylene*2 | toluene | 1.0 | benzoyl peroxide | (1.0) |
| 502 | butadiene-styrene copolymer*3 | " | 2.0 | " | (1.0) |
| 503 | polyurethane polyester*4 | " | 1.0 | triphenylmethane triisocyanate | (0.5) |

Note: *1: Per 100 parts by weight of the elastomeric polymer.

*2: Oppanol B100® produced by GASF

*3: Buna S® produced by Bayer A.G.

*4: Pandex produced by Dai-Nippon Ink & Chemicals, Inc.

TABLE 6

| Gas Permeability of Composite Membrane | | | | | | |
|---|---|---|---|---|---|---|
| Sample No. | Before Solvent Immersion | | | After Solvent Immersion | | |
| | $P(CO_2)$ | | α | $P(CO_2)$ | | α |
| | ($Nm^3/m^2$•hr•atm) | $Nm^3/m^2$•s•MPa | | ($Nm^3/m^2$•hr•atm) | $Nm^3/m^2$•s•MPa | |
| 501 | 0.8 x $10^{-1}$ | 28.2 | 69 | 0.8 x $10^{-1}$ | 28.2 | 69 |
| 502 | 1.8 x $10^{-1}$ | 63.5 | 150 | 1.8 x $10^{-1}$ | 63.5 | 147 |
| 503 | 1.5 x $10^{-1}$ | 56.5 | 88 | 1.6 x $10^{-1}$ | 56.5 | 90 |
| A | 1.0 x $10^{-1}$ | 35.3 | 73 | 0.8 x $10^{-1}$ | 28.2 | 21 |
| B | 2.0 x $10^{-1}$ | 70.6 | 113 | 0.5 x $10^{-1}$ | 17.7 | 42 |
| C | 2.1 x $10^{-1}$ | 74.1 | 61 | 1.2 x $10^{-1}$ | 42.4 | 15 |

EXAMPLE 6

A 1.0% cyclohexane solution of poly(4-methylpentene-1) was coated on the dense layer of the same supporting membrane as used in Example 5 to a thickness of 30 µm and dried at 120°C for 10 min to prepare a composite membrane having an elastomeric polymer film.

Then, the elastomeric polymer film of the composite membrane was irradiated with 0.5 x $10^4$ J/kg (0.5 Mrad) of an electron beam to crosslink the poly(4-methylpentene-1).

Each of the non-irradiated and irradiated composite membranes was immersed first in petroleum benzene and then in gasoline, washed with hexane, and dried in the same manner as in Example 5.

Each of the non-irradiated and irradiated membranes was determined for $P(CO_2)$ and α before and after the immersion in solvents, and the results obtained are shown in Table 7 below.

TABLE 7

| Treatment | Before Immersion | | | After Immersion | | |
|---|---|---|---|---|---|---|
| | PO(CO ) | | α | PO(CO ) | | α |
| | ($Nm^3/m^2$•hr•ato) | $Nm^3/m^2$•s•MPa | | ($Nm^3/m^2$•hr•ato | $Nm^3/m^2$•s•MPa | |
| Irradiated | 1.2 x $10^{-1}$ | 42.4 | 153 | 1.2 x $10^{-1}$ | 42.4 | 148 |
| Non-irradiated | 2.2 x $10^{-1}$ | 77.7 | 141 | 3.3 x $10^{-1}$ | 116.5 | 43 |

EXAMPLE 7

The same polyimide resin solution as used in Example 1 was prepared, and in the solution were dissolved 50 parts of dioxane (flocculation value: 170) as a swelling agent per 100 parts of the polyimide to prepare a film-forming solution.

The film-forming solution was coated on a polyester non-woven cloth to a thickness of 200 µm, and the cloth was dipped in isopropanol at 3°C for 10 s and then in water at 3°C to obtain a water-containing membrane having an anisotropic structure.

The resulting water-containing membranes was soaked first in ethanol and then in hexane for 3 h, respectively, followed by air-drying at 25°C to obtain a dried supporting membrane.

The solution of a crosslinkable silicone resin shown in Table 8 below was coated on the supporting membrane to a thickness of 50 µm, dried at 80°C for 10 min, and allowed to stand at room temperature for 2 days to obtain a composite membrane (Sample Nos. 701 to 705).

Each of the resulting composite membranes was determined for rate of permeation to carbon dioxide and coefficient of $CO_2/CH_4$ separation, and the results are shown in Table 8.

EXAMPLE 8

A solution of crosslinkable silicone resin shown in Table 8 was coated on the same supporting membrane as used in Example 7 to a thickness of 50 µm. After the solvent was removed by drying, the coating film was covered with a polyethylene sheet for the purpose of conducting crosslinking effectively. The film was then irradiated with $10^5$ J/kg (10 Mrad) of an electron beam in a nitrogen atmosphere to crosslink the silicone resin to obtain a composite membrane having a crosslinked silicone resin film (Sample Nos. 801 to 802).

Each of the resulting composite membranes was determined for rate of permeation to carbon dioxide and coefficient of $CO_2/CH_4$ sepration, and the results are shown in Table 8.

EXAMPLE 9

A 4.0% isooctane solution of one-can type RTV polydimethylsiloxane 615 was prepared, and benzoyl peroxide was added thereto. The solution was coatd on the same supporting membrane as used in Example 7 to a thickness of 50 µm and heated at 100 °C for 5 h to crosslink the silicone resin though methylene crosslinking reaction to obtain a composite membrane (Sample No. 901). The rate of permeation to carbon dioxide and coefficient of $CO_2/CH_4$ separation of Sample 901 are shown in Table 8.

TABLE 8

| Sample No. | Silicone Resin Solution: Silicone Resin | Silicone Resin Solution: Solvent | Silicone Resin Solution: Concn. (wt%) | Coefficient of $CO_2/CH_4$ Separation of Silicone Resin | Composite Membrane: Rate of Permeation to Carbon Dioxide ($Nm^3/m^2 \cdot hr \cdot atm$) $Nm^3/m^2 \cdot s \cdot MPa$ | Composite Membrane: Coefficient of $CO_2/CH_4$ Separation |
|---|---|---|---|---|---|---|
| 701 | SH-780*1 | ethyl acetate | 1.0 | 4.5 | 56.5 $1.6 \times 10^{-1}$ | 36 |
| 702 | SH-780 | " | 4.0 | 4.5 | 38.8 $1.1 \times 10^{-1}$ | 51 |
| 703 | RTV-615*2 | isooctane | 4.0 | 4.5 | 53.0 $1.5 \times 10^{-1}$ | 62 |
| 704 | SE-9155*3 | " | 4.0 | 4.5 | 53.0 $1.5 \times 10^{-1}$ | 57 |
| 705 | polydimethylsiloxane *4 | methyl acetate | 5.0 | 4.5 | 201.2 $5.7 \times 10^{-1}$ | 52 |
| 801 | SH-780 | ethyl acetate | 4.0 | 4.5 | 53.0 $1.5 \times 10^{-1}$ | 59 |
| 802 | SB-9155 | " | 4.0 | 4.5 | 63.5 $1.8 \times 10^{-1}$ | 62 |
| 901 | RTV-615 | isooctane | 4.0 | 4.5 | 77.7 $2.2 \times 10^{-1}$ | 71 |

Note:
*1: made by Toshiba Silicone Co., Ltd.
*2: made by General Electric Inc.
*3: made by Toray Silicone Co., Ltd.
*4: water-induced crosslinking type (de-oxime type); curing condition: heating at 60°C for 30 mins. and allowing to stand at room temperature for 1 day.

REFERENCE EXAMPLE

In a 500 mℓ-volume separable flask equipped with a stirrer, a thermometer, and side tube with a condenser were charged 46.8 g (0.20 mol) of butanetetracarboxylic acid and 0.20 mol of each of a diamine having the formula:

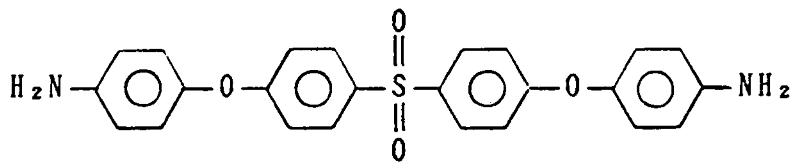

a diamine having the formula:

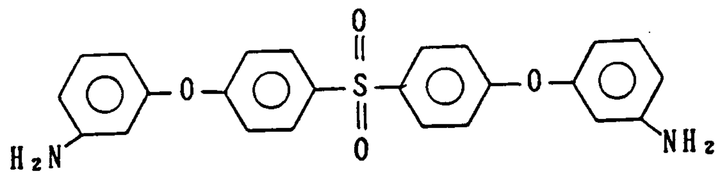

and a diamine having the formula:

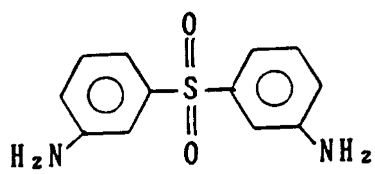

To the mixture were added 270 mℓ of N-methyl-2-pyrrolidone and 50 mℓ of xylene to dissolve the solid substances. The solution was heated at 180 °C for 30 to 40 min under thoroughly stirring to effect a condensation reaction while removing water as the xylene azeotrope. Thereafter, the xylene was completely removed by distillation to obtain an N-methyl-2-pyrrolidone solution of the respective polyimide resin, which was used in Example 10, 11, or 12, respectively.

EXAMPLE 10

A 18% N-methyl-2-pyrrolidone solution of a polyimide resin having a repeating unit of formula:

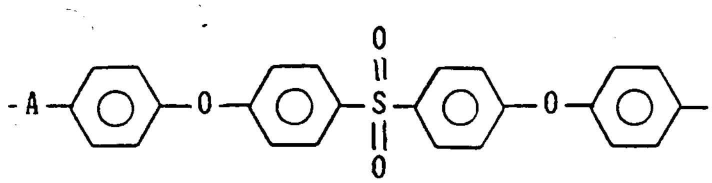

wherein A represents

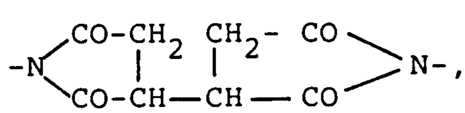

as prepared in Reference Example was coated on a polyester non-woven cloth to a thickness of 200 µm. The coated cloth was dipped in an organic solvent as shown in Table 9 under prescribed conditions and

then in water at 3 ° C to obtain a water-containing membrane having an anisotropic structure.

Each of the water-containing membranes was soaked in ethanol and then in hexane for 3 h, respectively, followed by air-drying at 25 ° C.

Each of the resulting dried membranes (Sample Nos. 1001 to 1020) was determined for rate of permeation to carbon dioxide and coefficient of $CO_2/CH_4$ separation, and the results are shown in Table 9.

EXAMPLE 11

A membrane was prepared in the same manner as in Example 10, except for using an N-methyl-2-pyrrolidone solution of polyimide-sulfone having a repeating unit of formula:

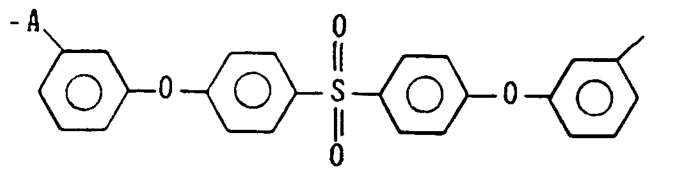

wherein A represents

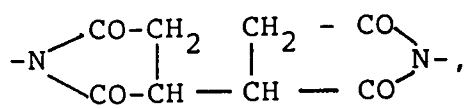

(intrinsic viscosity [η] : 0.51 dℓ/g) as prepared in Reference Example.

Each of the resulting membranes (Sample Nos. 1101 to 1108) was determined for rate of permeation to carbon dioxide and coefficient of $CO_2/CH_4$ separation, and the results are shown in Table 9.

EXAMPLE 12

A membrane was prepared in the same manner as in Example 10, except for using an N-methyl-2-pyrrolidone solution of a polyimide resin having a repeating unit of formula:

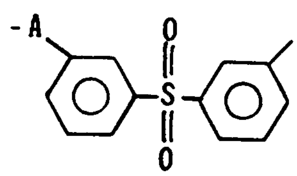

wherein A represents

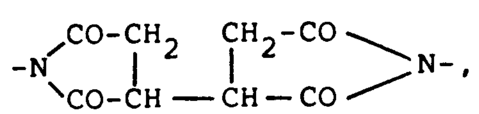

as prepared in Reference Example.

Each of the resulting membranes (Sample Nos. 1201 to 1208) was determined for rate of permeation to carbon dioxide and coefficient of $CO_2/CH_4$ separation, and the results are shown in Table 9.

TABLE 9

| Sample No. | Solvent Dipping Conditions: Organic Solvent | Temp. (°C) | Time (s~~ec~~) | Rate of Permeation to Carbon Dioxide ((Nm3/m2.hr.atm)) | Nm³/m²·s·MPa | Coefficient of CO2/CH4 Separation |
|---|---|---|---|---|---|---|
| 1001 | isopropyl alcohol | 3 | 10 | 0.61 | 215.3 | 56 |
| 1002 | " | " | 30 | 0.34 | 120.0 | 41 |
| 1003 | " | 20 | 10 | 0.57 | 201.2 | 41 |
| 1004 | " | " | 30 | 0.42 | 148.3 | 49 |
| 1005 | ethylene glycol | 3 | 10 | 9.9 | $34.9\times10^2$ | 5 |
| 1006 | " | " | 30 | 6.9 | $24.4\times10^2$ | 12 |
| 1007 | " | 20 | 10 | 3.4 | $12.0\times10^2$ | 18 |
| 1008 | " | " | 30 | 2.3 | 811.9 | 15 |
| 1009 | tetrahydrofuran | 3 | 10 | 8.6 | $30.4\times10^2$ | 7 |
| 1010 | " | " | 30 | 4.1 | $14.5\times10^2$ | 13 |
| 1011 | " | 20 | 10 | 7.8 | $27.5\times10^2$ | 17 |
| 1012 | " | " | 30 | 3.5 | $12.4\times10^2$ | 21 |
| 1013 | acetone | 3 | 10 | 5.4 | $19.1\times10^2$ | 26 |
| 1014 | " | " | 30 | 3.2 | $11.3\times10^2$ | 20 |
| 1015 | " | 20 | 10 | 4.1 | $14.3\times10^2$ | 22 |
| 1016 | " | " | 30 | 2.4 | 847.2 | 19 |
| 1017 | t-butanol | 25 | 10 | 2.2 | 776.6 | 35 |
| 1018 | " | " | 30 | 1.6 | 564.8 | 31 |
| 1019 | " | 45 | 3 | 1.9 | 670.7 | 19 |
| 1020 | " | " | 10 | 1.1 | 388.3 | 35 |

/To be cont'd.

TABLE 9 (cont'd.)

| Sample No. | Solvent Dipping Conditions: Organic Solvent | Temp. (°C) | Time (sec) | Rate of Permeation to Carbon Dioxide (Nm3/m2.hr.atm) | ($Nm^3/m^2 \cdot s \cdot MPa$) | Coefficient of CO2/CH4 Separation |
|---|---|---|---|---|---|---|
| 1101 | acetone | 3 | 10 | 8.3 | $29.3 \times 10^2$ | 12 |
| 1102 | " | " | 30 | 5.5 | $19.4 \times 10^2$ | 14 |
| 1103 | " | 20 | 10 | 6.7 | $23.7 \times 10^2$ | 19 |
| 1104 | " | " | 30 | 4.3 | $15.2 \times 10^2$ | 27 |
| 1105 | t-butanol | 25 | 10 | 3.4 | $12.0 \times 10^2$ | 19 |
| 1106 | " | " | 30 | 2.3 | $0.81 \times 10^2$ | 28 |
| 1107 | " | 45 | 3 | 2.8 | $0.98 \times 10^2$ | 39 |
| 1108 | " | " | 10 | 1.4 | $0.49 \times 10^2$ | 42 |
| 1201 | acetone | 3 | 10 | 10.2 | $36.0 \times 10^2$ | 1.2 |
| 1202 | " | " | 30 | 7.6 | $26.8 \times 10^2$ | 1.3 |
| 1203 | " | 20 | 10 | 8.2 | $28.9 \times 10^2$ | 11 |
| 1204 | " | " | 30 | 4.0 | $14.1 \times 10^2$ | 4 |
| 1205 | t-butanol | 25 | 10 | 5.0 | $17.7 \times 10^2$ | 8 |
| 1206 | " | " | 30 | 1.8 | $63.5 \times 10^2$ | 6 |
| 1207 | " | 45 | 3 | 4.9 | $17.3 \times 10^2$ | 13 |
| 1208 | " | " | 10 | 2.0 | $0.71 \times 10^2$ | 5 |

## EXAMPLE 13

A membrane was prepared in the same manner as Sample 1019 of Example 10, except that the polyimide resin solution further contained a swelling agent as shown in Table 10. Each of the resulting membranes (Sample Nos. 1301 to 1312) was determined for rate of permeation to carbon dioxide and coefficient of $CO_2/CH_4$ separation, and the results obtained are shown in Table 10.

TABLE 10

| Sample No. | Swelling Agent: Kind | Swelling Agent: Flocculation Value | Amount Added (part*) | Rate of Permeation to Carbon Dioxide ($Nm^3/m^2.hr.atm$) | ($Nm^3/m^2 \cdot s \cdot MPa$) | Coefficient of CO2/CH4 Separation |
|---|---|---|---|---|---|---|
| 1301 | acetone | 63 | 25 | 2.5 | $0.88 \times 10^2$ | 63 |
| 1302 | " | " | 50 | 6.2 | $21.9 \times 10^2$ | 58 |
| 1303 | " | " | 100 | 4.3 | $15.2 \times 10^2$ | 42 |
| 1304 | " | " | 200 | 1.9 | $0.67 \times 10^2$ | 29 |
| 1305 | dioxane | 170 | 25 | 5.8 | $20.5 \times 10^2$ | 42 |
| 1306 | " | " | 50 | 10.2 | $36.0 \times 10^2$ | 39 |
| 1307 | " | " | 100 | 8.1 | $28.6 \times 10^2$ | 30 |
| 1308 | " | " | 200 | 6.0 | $21.2 \times 10^2$ | 27 |
| 1309 | tetrahydrofuran | 88 | 25 | 4.2 | $14.8 \times 10^2$ | 41 |
| 1310 | " | " | 50 | 7.9 | $27.9 \times 10^2$ | 40 |
| 1311 | " | " | 100 | 6.0 | $21.2 \times 10^2$ | 35 |
| 1312 | " | " | 200 | 4.2 | $14.8 \times 10^2$ | 32 |

Note: * per 100 parts by weight of the resin.

EXAMPLE 14

The same N-methyl-2-pyrrolidone solution of a polyimide resin as used in Example 10 was coated on a polyester non-woven cloth to a thickness of 200 μm. The cloth was dipped in t-butanol at 45 °C for 3 s and then in water at 3 °C to obtain a water-containing membrane having an anisotropic structure.

The water-containing membrane was soaked in ethanol and then in hexane for 3 h, respectively, and air-dried at 25 °C to obtain a dried membrane as a supporting membrane.

On the supporting membrane was coated an elastomeric polymer solution as shown in Table 11 to a thickness of 30 μm, followed by drying at 80 °C for 30 min to obtain a composite membrane.

Each of the resulting composite membranes (Sample Nos. 1401 to 1404) was determined for rate of permeation to carbon dioxide and coefficient of $CO_2/CH_4$ separation, and the results obtained are shown in Table 11.

TABLE 11

| Sample No. | Elastomeric Polymer Solution | | | Coefficient of CO2/CH4 Separation of Elastomeric Polymer | Composite Membrane | | |
|---|---|---|---|---|---|---|---|
| | Elastomeric Polymer | Solvent | Concn. (wt%) | | Nm³/m²·s·MPa | Rate of Permeation to Carbon Dioxide (Nm3/m2.hr.atm) | Coefficient of CO2/CH4 Separation |
| 1401 | poly(4-methylpentene-1) | cyclohexane | 1.0 | 5.4 | 388.3 | 1.10 | 192 |
| 1402 | butadiene-styrene copolymer | toluene | 1.0 | 18 | 324.8 | 0.92 | 133 |
| 1403 | polyisobutylene | isooctane | 0.5 | 11 | 194.2 | 0.55 | 94 |
| 1404 | isoprene-isobutylene copolymer | toluene | 1.0 | 12 | 296.5 | 0.84 | 62 |

EXAMPLE 15

Composite membranes (Sample Nos. 1501 to 1506) were prepared in the same manner as in Example 14, except for using each of the elastomeric polymer solutions shown in Table 12 below. In the preparation of Samples 1505 and 1506, the conditions for the film formation on the suporting membrane were altered according to the footnote of Table 12.

Each of the resulting composite membranes was determined for rate of permeation to carbon dioxide and coefficient of $CO_2/CH_4$ separation, and the results obtained are shown in Table 12.

TABLE 12

| Sample No. | Elastomeric Polymer Solution: Elastomeric Polymer | Solvent | Concn. (wt%) | Coefficient of CO2/CH4 Separation of Elastomeric Polymer | Composite Membrane: Rate of Permeation to Carbon Dioxide (Nm3/m2.hr.atm) | Nm³/m²·s·MPa | Coefficient of CO2/CH4 Separation |
|---|---|---|---|---|---|---|---|
| 1501 | polyester polyol | chloroform | 1.0 | 10 | 0.44 | 155.3 | 98 |
| 1502 | polyurethane polyol | tetrahydrofuran | 1.0 | 11 | 0.37 | 130.6 | 131 |
| 1503 | polyurethane polyester | " | 1.0 | 19 | 0.50 | 176.5 | 120 |
| 1504 | polyamide polyether | chloroform | 0.5 | 9 | 0.79 | 278.9 | 134 |
| 1505 | epoxy resin* | toluene | 5.0 | 15 | 0.12 | 42.4 | 155 |
| 1506 | ethyl cellulose** | chloroform | 2.0 | 18 | 0.68 | 240.0 | 134 |

Note: * : Comprised 100 parts of Epon 828® (made by Shell Chemical), 10 parts of a curing agent CIBN (carboxyl-terminated butadiene-acrylonitrile copolymer), and 5 parts of tetramethylguanidine; curing conditions: heating at 180°C for 1 h.

**: Heated at 60°C for 30 min.

EXAMPLE 16

A solution of an elastomeric polymer containing a radical generator or a polyisocyanate as a crosslinking agent as shown in Table 5 was coated on the same supporting membrane as used in Example

14 to a thickness of 30 µm and dried at 120 °C for 10 min to obtain a composite membrane having a crosslinked elastomeric polymer film.

Each of the resulting composite membranes (Sample Nos. 1601 to 1603) was determined for rate of permeation to carbon dioxide [$P(CO_2)$] and coefficient of $CO_2/CH_4$ separation (α), and the results obtained are shown in Table 13.

TABLE 13

| Sample No. | Gas Permeability Before Immersion in Solvent | | | Gas Permeability After Immersion in Solvent | | |
|---|---|---|---|---|---|---|
| | P(CO ) | | α | P(CO ) | | α |
| | ($Nm^3/m^2$• hr•atm) | $Nm^3/m^2$•s•MPa | | ($Nm^3/m^2$• hr•atm) | $Nm^3/m^2$•s•MPa | |
| 1601 | 0.88 | 310.6 | 67 | 0.92 | 324.8 | 71 |
| 1602 | 1.08 | 381.2 | 152 | 1.11 | 391.8 | 145 |
| 1603 | 1.22 | 430.7 | 90 | 1.02 | 360.1 | 92 |

EXAMPLE 17

A 1.0% solution of poly(4-methylpentene-1) in cyclohexane was prepared. The solution was coated on the same supporting membane as used in Example 14 to a thickness of 30 µm and dried at 120 °C for 10 min to obtain a composite membrane having an elastomeric polymer film.

The elastomeric polymer film was irradiated with an electron beam of 0.5 x $10^4$ J/kg (0.5 Mrad) in a nitrogen atmosphere to crosslink the poly(4-methylpentene-1).

Each of the non-irradiatd composite membrane and the irradiated composite membrane was determined for rate of permeation to carbon dioxide and coefficient of $CO_2/CH_4$ separation, and the results obtained are shown in Table 14.

Then, each of the membranes was immersed in petroleum benzine and then in gasoline, washed with hexane, and dried in the same manner as in Example 16. The thus treated membrane was determined for rate of permeation to carbon dioxide and coefficient of $CO_2/CH_4$ separation, and the results obtained are shown in Table 14.

TABLE 14

| Sample | Gas Permeability Before Immersion in Solvent | | | Gas Permeability After Immersion in Solvent | | |
|---|---|---|---|---|---|---|
| | P(CO ) | | α | P(CO ) | | α |
| | ($Nm^3/m^2$• hr•atm) | $Nm^3/m^2$•s•MPa | | ($Nm^3/m^2$• hr•atm) | $Nm^3/m^2$•s•MPa | |
| Irradiated | 0.68 | 240.0 | 161 | 0.76 | 268.3 | 152 |
| Non-irradiated | 1.31 | 462.4 | 139 | 1.33 | 469.5 | 52 |

EXAMPLE 18

A crosslinkable silicone resin solution shown in Table 15 was coated on the same supporting membrane as used in Example 14 to a thickness of 50 µm, dried at 80 °C for 10 min, and allowed to stand at room temperature for 2 days to obtain a composite membrane (Sample Nos. 1801 to 1805).

Each of the composite membranes was determined for rate of permeation to carbon dioxide and coefficient of $CO_2/CH_4$ separation, and the results obtained are shown in Table 15.

EXAMPLE 19

A crosslinkable silicone resin solution shown in Table 15 was coated on the same supporting membrane as used in Example 14 to a thickness of 50 µm and dried to remove the solvent. In order to conduct crosslinking efficiently, the silicone resin film was covered with a polyethylene sheet, and the film was irradiated with an electron beam $10^5$ J/kg of (10 Mrad) in a nitrogen atmosphere to crosslink the silicone resin to obtain a composite membrane (Sample Nos. 1901 to 1902).

Each of the composite membranes was determined for rate of permeation to carbon dioxide and coefficient of $CO_2/CH_4$ separation, and the results obtained are shown in Table 15.

EXAMPLE 20

An isooctane solution of one-can type RTV polydimethylsiloxane 615 as shown in Table 15 was prepared, and benzoyl peroxide was added thereto. The resulting solution was coated on the same supporting membrane as used in Example 14 to a thickness of 50 µm and heated at 100 °C for 5 h to crosslink the silicone resin to obtain a composite membrane (Sample No. 2001).

The resulting composite membrane was determined for rate of permeation to carbon dioxide and coefficient of $CO_2/CH_4$ separation, and the results are shown in Table 15.

TABLE 15

| Sample No. | Silicone Resin Solution: Silicone Resin | Silicone Resin Solution: Solvent | Silicone Resin Solution: Concn. (wt%) | Coefficient of $CO_2/CH_4$ Separation of Silicone Resin | Nm³/m²·s·MPa | Composite Membrane: Rate of Permeation to Carbon dioxide ($Nm^3/m^2 \cdot hr \cdot atm$) | Composite Membrane: Coefficient of $CO_2/CH_4$ Separation |
|---|---|---|---|---|---|---|---|
| 1801 | SH-780 *1 | ethyl acetate | 1.0 | 4.5 | 151.8 | 0.43 | 84 |
| 1802 | SH-780 | " | 4.0 | 4.5 | 131.1 | 0.38 | 91 |
| 1803 | RTV-615 *2 | isooctane | 4.0 | 4.5 | 197.7 | 0.56 | 96 |
| 1804 | SE-9155 *3 | " | 4.0 | 4.5 | 190.6 | 0.54 | 102 |
| 1805 | polydimethylsiloxane *4 | methyl acetate | 5.0 | 4.5 | 285.9 | 0.81 | 125 |
| 1901 | SH-780 | ethyl acetate | 4.0 | 4.5 | 233.0 | 0.66 | 69 |
| 1902 | SE-9155 | " | 4.0 | 4.5 | 278.9 | 0.79 | 78 |
| 2001 | RTV-615 | isooctane | 4.0 | 4.5 | 225.9 | 0.64 | 92 |

Note:
*1: made by Toshiba Silicone Co., Ltd.
*2: made by General Electric Inc.
*3: made by Toray Silicone Co., Ltd.
*4: water-induced crosslinking type (de-oxime type); curing condition: heating at 60°C for 30 min. and allowing to stand at room temperature for 1 day.

EXAMPLE 21

A dried membrane was prepared in the same manner as in Example 14, except for using the same polyimide resin as used in Example 11. A 1% solution of poly(4-methylpentene-1) was coated on the dense layer of the dried membrane to a thickness of 30 µm and dried at 80 °C for 30 min to obtain a composite membrane (Sample No. 2101).

A composite membrane was prepared in the same manner as described above, except for using the same polyimide resin as used in Example 12 (Sample No. 2102).

Each of the resulting composite membranes was determined for rate of permeation to carbon dioxide and coefficient of $CO_2/CH_4$ separation, and the results obtained are shown in Table 16.

TABLE 16

| Sample No. | Gas Permeability P(SO ) (Nm3/m2.hr.atm) Nm³/m²·s·MPa | α |
|---|---|---|
| 2101 | 183.6 (0.52) | 129 |
| 2002 | 127.1 (0.36) | 99 |

## Claims

1. A process for separating methane from a gaseous mixture containing methane and carbon dioxide by concentration, which comprises contacting said gaseous mixture with a membrane comprising a film of a polyimide resin having a repeating unit represented by formula (I):

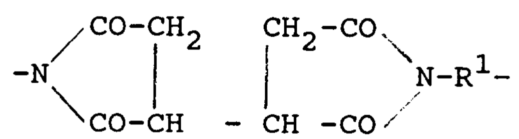

(I)

wherein $R^1$ represents a divalent aromatic, alicyclic or aliphatic hydrocarbon group, or a divalent organic group composed of these hydrocarbon groups linked via a divalent organic linking group,
to selectively pass carbon dioxide through said membrane.

2. The process as claimed in claim 1, wherein $R^1$ represents an aromatic hydrocarbon group or an aromatic hydrocarbon group composed of two aromatic hydrocarbon groups connected by an organic linking group.

3. The process as claimed in claim 2, wherein $R^1$ represents an aromatic hydrocarbon group composed of two aromatic hydrocarbon groups connected via an alkylene group or an ether group.

4. The process as claimed in claim 3, wherein $R^1$ is

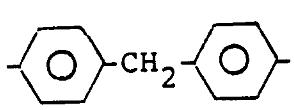 or 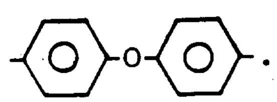.

5. The process as claimed in claim 1, wherein $R^1$ represents a divalent organic group containing a sulfo group represented by formula (II):

$-R^2-SO_2-R^3-$ (II)

wherein $R^2$ and $R^3$ each represents a divalent aromatic, alicyclic or aliphatic hydrocarbon group, or a divalent organic group composed of these hydrocarbon groups via a divalent organic linking group.

6. The process as claimed in claim 5, wherein both $R^2$ and $R^3$ represent an aromatic hydrocarbon group or an aromatic hydrocarbon group composed of two aromatic hydrocarbon groups linked via an organic linking group.

7. The process as claimed in claim 5, wherein $R^1$ is

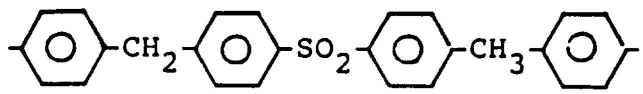,

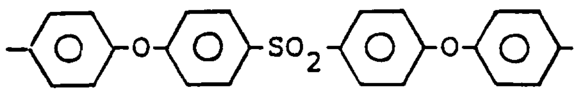 or 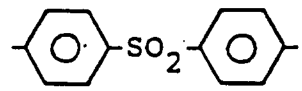.

8. The process as claimed in claim 1, wherein said polyimide resin has an intrinsic viscosity [$\eta$] of from 0.4 to 2 dl/g as measured in N-methyl-2-pyrrolidone at 30 ° C.

9. The process as claimed in claim 1, wherein said film of a polyimide resin is obtained by a process comprising preparing a film-forming solution comprised of the polyimide resin having the repeating unit of formula (I), a water-miscible organic solvent, and at least one liquid swelling agent having a flocculation value for said polyimide resin in the range of from 50 to 200 and having a boiling point of from 50 to 120 ° C at atmospheric pressure which is selected from the group consisting of cyclic ethers, aliphatic ketones, alicyclic ketones, lower aliphatic carboxylic acids, and lower aliphatic carboxylic acid lower alkyl esters, the flocculation value of said swelling agent for the polyimide resin being a minimum number of milliliters of the swelling agent necessary to make 50 ml of a 2 wt% solution of the polyimide resin in N-methylpyrrolidone become white turbid at 25 ° C due to flocculation of the resin, coating the film-forming solution on a base, dipping the coated base in an organic solvent incapable of disssolving said polyimide resin but miscible with either of said water-miscible organic solvent and water and then in water to obtain a water-containing polyimide membrane having an anisotropic structure, and drying the polyimide membrane.

10. The process as claimed in claim 9, wherein said water-miscible organic solvent is selected from the group consisting of an N-alkyl-2-pyrrolidone, an N-alkyl-2-piperidone, a dialkylacetamide, and a dialkylformamide.

11. The process as claimed in claim 9, wherein said water-miscible organic solvent is N-methyl-2-pyrrolidone.

12. The process as claimed in claim 9, wherein said solvent incapable of dissolving the polyimide resin but miscible with either of the water-miscible organic solvent and water is selected from the group consisting of lower aliphatic alcohols, alcohols having a cyclic structure, ketones, esters of formic acid, acetic acid, lactic acid or phosphoric acid, polyhydric alcohols and derivatives thereof, ethers and nitriles.

13. The process as claimed in claim 9, wherein said dipping in the organic solvent is carried out at a temperature of from 0 ° to 100 ° C for 5 min or less.

14. The process as claimed in claim 9, wherein said swelling agent is selected from the group consisting of tetrahydrofuran, dioxane, acetone, methyl ethyl ketone, diethyl ketone, cyclohexanone, acetic acid, formic acid, methyl formate, ethyl formate and methyl acetate.

15. The process as claimed in claim 9, wherein said swelling agent is used in an amount of from 30 to 300 parts by weight per 100 parts by weight of the polyimide resin.

16. The process as claimed in claim 1, wherein said membrane further comprises an elastomeric polymer film formed on said polyimide resin membrane.

17. The process as claimed in claim 16, wherein said polyimide resin film has a coefficient of $CO_2/CH_4$ separation of at least 0.7.

18. The process as claimed in claim 16, wherein said elastomeric polymer film has a coefficient of $CO_2/CH_4$ separation of at least 3 and a softening point of at least 50 °C.

19. The process as claimed in claim 16, wherein said elastomeric polymer film is a crosslinked elastomeric polymer film.

20. The process as claimed in claim 19, wherein said crosslinked elastomeric polymer film is formed by crosslinking a crosslinkable silicone resin.

21. The process as claimed in claim 20, wherein said crosslinkable silicone resin has an average molecular weight of from 10,000 to 300,000.

**Patentansprüche**

1. Verfahren zur Abtrennung von Methan aus einem Gasgemisch, enthaltend Methan und Kohlendioxid, durch Konzentrieren, welches das Zusammenbringen des Gasgemisches mit einer Membran, umfassend einen Film eines Polyimidharzes mit einer sich wiederholenden Einheit der Formel (I):

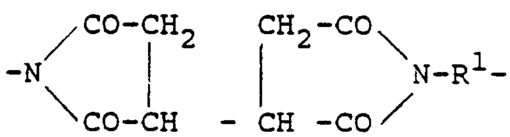

(I)

umfaßt, worin $R^1$ eine zweiwertige aromatische, alicyclische oder aliphatische Kohlenwasserstoffgruppe oder eine zweiwertige organische Gruppe, zusammengesetzt aus diesen Kohlenwasserstoffgruppen, die über eine zweiwertige organische Verbindungsgruppe verknüpft sind, darstellt, um das Kohlendioxid selektiv durch die Membran zu leiten.

2. Verfahren nach Anspruch 1, worin $R^1$ eine aromatische Kohlenwasserstoffgruppe oder eine aromatische Kohlenwasserstoffgruppe, zusammengesetzt aus zwei aromatischen Kohlenwasserstoffgruppen, die durch eine organische Verbindungsgruppe verbunden sind, ist.

3. Verfahren nach Anspruch 2, worin $R^1$ eine aromatische Kohlenwasserstoffgruppe, zusammengesetzt aus zwei aromatischen Kohlenwasserstoffgruppen, die über eine Alkylengruppe oder eine Ethergruppe verbunden sind, ist.

4. Verfahren nach Anspruch 3, worin $R^1$

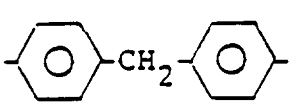

oder

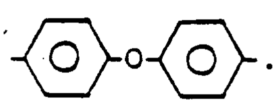

ist.

5. Verfahren nach Anspruch 1, worin $R^1$ eine zweiwertige organische Gruppe, enthaltend eine Sulfogruppe, dargestellt durch die Formel (II):

$-R^2-SO_2-R^3-$ (II)

ist, worin $R^2$ und $R^3$ jeweils eine zweiwertige aromatische, alicyclische oder aliphatische Kohlenwasserstoffgruppe oder eine zweiwertige organische Gruppe, zusammengesetzt aus diesen Kohlenwasserstoffgruppen über eine zweiwertige organische Verbindungsgruppe, darstellen.

6. Verfahren nach Anspruch 5, worin sowohl $R^2$ als auch $R^3$ eine aromatische Kohlenwasserstoffgruppe oder eine aromatische Kohlenwasserstoffgruppe, zusammengesetzt aus zwei aromatischen Kohlenwasserstoffgruppen, die über eine organische Verbindungsgruppe verknüpft sind, ist.

7. Verfahren nach Anspruch 5, worin $R^1$

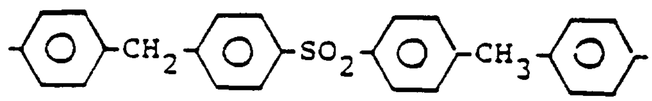,

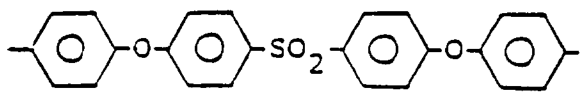 oder 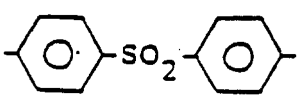.

ist.

8. Verfahren nach Anspruch 1, worin das Polyimidharz eine Strukturviskosität [$\eta$] von 0,4 bis 2 dl/g, gemessen in N-Methyl-2-pyrrolidon bei 30° C, aufweist.

9. Verfahren nach Anspruch 1, worin der Film aus einem Polyimidharz durch ein Verfahren erhalten wird, das die folgenden Stufen umfaßt:
Herstellen einer Schichtbildenden Lösung, umfassend das Polyimidharz mit der sich wiederholenden Einheit der Formel (I), ein wassermischbares organisches Lösungsmittel und mindestens ein flüssiges Quellmittel mit einem Ausflockungswert für das Polyimidharz in dem Bereich von 50 bis 200 und einem Siedepunkt von 50 bis 120° C unter atmosphärischem Druck, ausgewählt aus der Gruppe, bestehend aus cyclischen Ethern, aliphatischen Ketonen, alicyclischen Ketonen, niedrigen aliphatischen Carbonsäuren und niedrigen Alkylestern niedriger aliphatischer Carbonsäuren, wobei der Ausflockungswert des Quellmittels für das Polyimidharz die minimale Anzahl von Millilitern ist, die nötig ist, um 50 ml einer 2 Gew.-%-igen Lösung des Polyimidharzes in N-Methylpyrrolidon bei 25° C aufgrund der Ausflockung des Harzes trüb-weiß zu machen,
Auftragen der Schichtbildenden Lösung auf eine Grundlage,
Eintauchen der beschichteten Grundlage in ein organisches Lösungsmittel, das unfähig ist, das Polyimidharz aufzulösen, aber mit sowohl dem wassermischbaren organischen Lösungsmittel als auch mit Wasser vermischbar ist, und dann in Wasser, um eine wasserhaltige Polyimidmembran mit einer anisotropen Struktur zu erhalten,
und Trocknen der Polyimidmembran.

10. Verfahren nach Anspruch 9, worin das wassermischbare organische Lösungsmittel ausgewählt ist aus der Gruppe, bestehend aus einem N-Alkyl-2-pyrrolidon, einem N-Alkyl-2-piperidon, einem Dialkylacetamid und einem Dialkylformamid.

11. Verfahren nach Anspruch 9, worin das wassermischbare organische Lösungsmittel in N-Methyl-2-pyrrolidon ist.

12. Verfahren nach Anspruch 9, worin das Lösungsmittel, das unfähig ist, das Polyimidharz aufzulösen, aber mit sowohl dem wassermischbaren organischen Lösungsmittel als auch mit Wasser mischbar ist, ausgewählt ist aus der Gruppe, bestehend aus niedrigen aliphatischen Alkoholen, Alkoholen mit

cyclischer Struktur, Ketonen, Estern von Ameisensäure, Essigsäure, Milchsäure oder Phosphorsäure, mehrwertigen Alkoholen und Derivaten davon, Ethern und Nitrilen.

**13.** Verfahren nach Anspruch 9, worin das Eintauchen in das organische Lösungsmittel bei einer Temperatur von 0 bis 100° C 5 min lang oder weniger durchgeführt wird.

**14.** Verfahren nach Anspruch 9, worin das Quellmittel ausgewählt ist aus der Gruppe, bestehend aus Tetrahydrofuran, Dioxan, Aceton, Methylethylketon, Diethylketon, Cyclohexanon, Essigsäure, Ameisensäure, Methylformiat, Ethylformiat udn Methylacetat.

**15.** Verfahren nach Anspruch 9, worin das Quellmittel in einer Menge von 30 bis 300 Gewichtsteilen pro 100 Gewichtsteile des Polyimidharzes verwendet wird.

**16.** Verfahren nach Anspruch 1, worin die Membran weiterhin eine auf der Polyimidharzmembran gebildete Elastomerschicht umfaßt.

**17.** Verfahren nach Anspruch 16, worin die Polyimidharzschicht einen $CO_2/CH_4$ Trennungskoeffizienten von mindestens 0,7 aufweist.

**18.** Verfahren nach Anspruch 16, worin die Elastomerschicht einen $CO_2/CH_4$ Trennungskoeffizienten von mindestens 3 und einen Erweichungspunkt von mindestens 50° C aufweist.

**19.** Verfahren nach Anspruch 16, worin die Elastomerschicht eine vernetzte Elastomerschicht ist.

**20.** Verfahren nach Anspruch 19, worin die vernetzte Elastomerschicht durch Vernetzen eines vernetzbaren Silikonharzes gebildet ist.

**21.** Verfahren nach Anspruch 20, worin das vernetzbare Silikonharz ein durchschnittliches Molekulargewicht von 10000 bis 300000 aufweist.

## Revendications

**1.** Un procédé de séparation du méthane d'un mélange gazeux contenant du méthane et du dioxyde de carbone par concentration, qui comprend la mise en contact dudit mélange gazeux avec une membrane comprenant un film d'une résine de polyimide ayant une unité de récurrence représentée par la formule (I) :

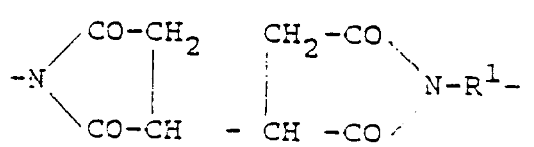

(I)

dans laquelle $R^1$ représente un groupe hydrocarboné divalent aromatique, alicyclique ou aliphatique, ou un groupe organique divalent composé de ces groupes hydrocarbonés liés via un groupe de liaison organique divalent, pour faire passer sélectivement du dioxyde de carbone à travers ladite membrane.

**2.** Le procédé selon la revendication 1, selon laquelle $R^1$ représente un groupe hydrocarboné aromatique ou un groupe hydrocarboné aromatique composé de deux groupes hydrocarbonés aromatiques reliés par un groupe de liaison organique.

**3.** Le procédé selon la revendication 2, selon laquelle $R^1$ représente un groupe hydrocarboné aromatique composé de deux groupes hydrocarbonés aromatiques reliés par un groupe alkylène ou un groupe éther.

**4.** Le procédé selon la revendication 3, selon laquelle $R^1$ est :

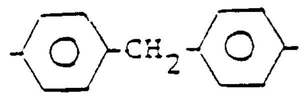 ou 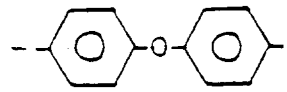 .

**5.** Le procédé selon la revendication 1, selon laquelle $R^1$ représente un groupe organique divalent contenant un groupe sulfo représenté par la formule (II):

$-R^2-SO_2-R^3-$ (II)

dans laquelle $R^2$ et $R^3$ représentent chacun un groupe hydrocarboné divalent aromatique, alicyclique ou aliphatique, ou un groupe organique divalent composé de ces groupes hydrocarbonés via un groupe de liaison organique divalent.

**6.** Le procédé selon la revendication 5, selon laquelle $R^2$ et $R^3$ représentent tous les deux un groupe hydrocarboné aromatique ou un groupe hydrocarboné aromatique composé de deux groupes hydrocarbonés aromatiques liés via un groupe de liaison organique.

**7.** Le procédé selon la revendication 5, selon laquelle $R^1$ est :

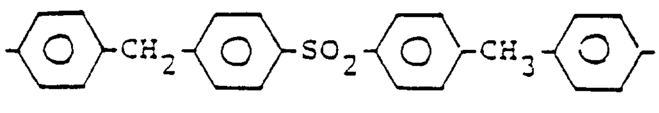 ,

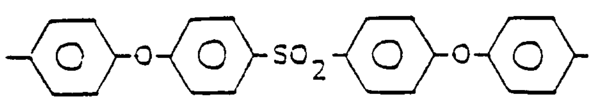 ou 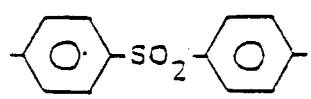 .

**8.** Le procédé selon la revendication 1, selon laquelle ladite résine de polyimide a une viscosité intrinsèque [$\eta$] de 0,4 à 2 dl/g mesurée dans la N-méthyl-2-pyrrolidone à 30 ° C.

**9.** Le procédé selon la revendication 1, selon laquelle ledit film d'une résine de polyimide est obtenu par un procédé comprenant la préparation d'une solution filmogène comprenant la résine de polyimide ayant l'unité de récurrence de formule (I), un solvant organique miscible à l'eau, et au moins un agent de gonflement liquide ayant une valeur de floculation pour ladite résine de polyimide dans l'intervalle de 50 à 200 et ayant un point d'ébullition de 50 à 120 ° C à la pression atmosphérique qui est choisi dans le groupe comprenant des éthers cycliques, des cétones aliphatiques, des cétones alicycliques, des acides carboxyliques aliphatiques inférieurs, et des esters alkyliques inférieurs d'acides carboxyliques aliphatiques inférieurs, la valeur de floculation dudit agent de gonflement pour la résine de polyimide étant le nombre minimal de ml de l'agent de gonflement nécessaire pour donner à 50 ml d'une solution à 2% en poids de la résine de polyimide dans la N-méthylpyrrolidone un trouble blanc à 25 ° C en raison de la floculation de la résine, le revêtement de la solution filmogène sur une base, le trempage dé la base revêtue dans un solvant organique incapable de dissoudre ladite résine polyimide mais miscible avec ce solvant organique miscible à l'eau et avec l'eau, et ensuite dans l'eau pour obtenir une membrane de polyimide contenant de l'eau ayant une structure anisotrope, et le séchage de la membrane de polyimide.

**10.** Le procédé selon la revendication 9, selon laquelle ledit solvant organique miscible à l'eau est sélectionné dans le groupe comprenant les suivants : une N-alkyl-2-pyrrolidone, une N-alkyl-2-pipéridone, un dialkylacétamide et un dialkylformamide.

**11.** Le procédé selon la revendication 9, selon laquelle ledit solvant organique miscible à l'eau est la N-méthyl-2-pyrrolidone.

**12.** Le procédé selon la revendication 9, selon laquelle ledit solvant incapable de dissoudre la résine de polyimide mais miscible avec le solvant organique miscible à l'eau et avec l'eau est sélectionné dans le groupe comprenant les suivants : les alcools aliphatiques inférieurs, les alcools ayant une structure cyclique, les cétones, les esters de l'acide formique, de l'acide acétique, de l'acide lactique ou de l'acide phosphorique, les alcools polyhydriques et leurs dérivés, les éthers et les nitriles.

**13.** Le procédé selon la revendication 9, selon laquelle le trempage dans ledit solvant organique est conduit à une température de 0° à 100°C pendant 5 min ou moins.

**14.** Un procédé selon la revendication 9, selon laquelle ledit agent de gonflement est choisi dans le groupe comprenant les suivants : tétrahydrofuranne, dioxanne, acétone, méthyléthylcétone, diéthylcétone, cyclohexanone, acide acétique, acide formique, formiate de méthyle, formiate d'éthyle, et acétate de méthyle.

**15.** Le procédé selon la revendication 9, selon laquelle ledit agent de gonflement est utilisé en quantité de 30 à 300 parties en poids pour 100 parties en poids de la résine de polyimide.

**16.** Le procédé selon la revendication 1, selon laquelle ladite membrane comprend également un film de polymère élastomère formé sur ladite membrane de résine de polyimide.

**17.** Un procédé selon la revendication 16, selon laquelle ledit film de résine de polyimide a un coefficient de séparation de $CO_2/CH_4$ d'au moins 0,7.

**18.** Le procédé selon la revendication 16, selon laquelle ledit film de polymère élastomère à un coefficient de séparation de $CO_2/CH_4$ d'au moins 3 et un point de ramollissement d'au moins 50°C.

**19.** Le procédé selon la revendication 16, selon laquelle ledit film de polymère élastomère est un film de polymère élastomère réticulé.

**20.** Le procédé selon la revendication 19, selon laquelle ledit film de polymère élastomère réticulé est formé par réticulation d'une résine de silicone réticulable.

**21.** Le procédé selon la revendication 20, selon laquelle ladite résine de silicone réticulable a un poids moléculaire moyen de 10 000 à 300 000.